(19) Europäisches Patentamt
European Patent Office
Office européen des brevets

(11) Numéro de publication: 0 176 396 B1

(12) **FASCICULE DE BREVET EUROPEEN**

(45) Date de publication du fascicule du brevet: 31.01.90

(51) Int. Cl.⁴: C 07 H 21/00, C 12 Q 1/68

(21) Numéro de dépôt: 85401665.6

(22) Date de dépôt: 21.08.85

(54) **Sondes comportant des groupements adénine modifiée, leur préparation et leurs utilisations.**

(30) Priorité: 22.08.84 FR 8413095

(43) Date de publication de la demande: 02.04.86 Bulletin 86/14

(45) Mention de la délivrance du brevet: 31.01.90 Bulletin 90/5

(84) Etats contractants désignés: AT BE CH DE GB IT LI LU NL SE

(56) Documents cités:
Biophysical Chemistry (1980), Tome III, R.C. Comptor et P.R: Schimmel, pages 1114 à 1116 et 1139 à 1147, ainsi que 1150 à 1160
CHEMICAL ABSTRACTS, vol. 94, no. 13, 30.03.1981, page 232, no. 97632j; Colubus, Ohio, US M. SPODHEIM MAURIZOT et al.: "Antibodies to N-hydroxy-2-aminofluorene modified DNA as probes in the study of DNA reacted with derivatives of 2-acetylaminofluorene."

(73) Titulaire: INSTITUT PASTEUR, 25-28, rue du Docteur Roux, F-75724 Paris Cédex 15 (FR)
Titulaire: Etablissement Public dit: CENTRE NATIONAL DE LA RECHERCHE SCIENTIFIQUE (CNRS), 15, Quai Anatole France, F-75007 Paris (FR)

(72) Inventeur: Huynh-Dinh, Tam, 32, rue Paul Déroulède, F-78290 Croissy sur Seine (FR)
Inventeur: Igolen, Jean, 4, rue Croix Mathurine Rodon, F-78320 Le Mesnil st Denis (FR)

(74) Mandataire: Peaucelle, Chantal et al, S.C. Ernest Gutmann - Yves Plasseraud 67, boulevard Haussmann, F-75008 Paris (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 176 396 B1

ACTORUM AG

## Description

L'invention concerne des sondes constituées de polynucléotides, plus précisément de fragments d'oligonucléotides, comportant, en tant que bases puriques, des groupements adénine modifiés de telle sorte qu'ils soient susceptibles de former trois liaisons hydrogène avec les groupes réactifs de la thymine, un procédé pour leur obtention ainsi que leurs applications.

Il est maintenant bien connu des spécialistes d'avoir recours pour certaines analyses, notamment pour la détection de séquences nucléotidiques particulières, telles que des séquences d'ADN ou d'ARN et l'isolement de telles séquences, à des techniques basées sur l'hybridation entre une séquence déterminée de nucléotides utilisée en tant que sonde et une séquence de nucléotides complémentaires de ceux de la sonde, éventuellement contenue dans une composition renfermant des acides nucléiques. La mise en œuvre de ces techniques, en particulier l'obtention de sondes sensibles et s'hybridant de façon stable avec les séquences complémentaires, pose encore des problèmes.

Ainsi, l'un des problèmes les plus fréquents posés en génie génétique est celui de la «pêche d'ARN messagers par des oligodésoxynucléotides synthétiques, c'est-à-dire la détection, l'isolement et l'analyse de séquences d'ARN messagers par hybridation avec des fragments d'ADN complémentaires (cADN). Cette technique est basée sur l'appariement des paires A-T (2 liaisons hydrogène) et G-C (3 liaisons hydrogène).

La conception et la synthèse de ces sondes sont rendues compliquées par la dégénérescence du code génétique: à part la méthionine (AUG) et la tryptophane (UGG) qui nécessitent un seul codon, tous les autres acides aminés sont traduits à partir de 2 à 6 codons.

Jusqu'à maintenant, à partir d'une séquence protéique déterminée, pour obtenir des sondes, on a utilisé plusieurs stratégies:

a) choisir dans une séquence G de préférence à A, et T de préférence à C, ce qui donne à l'hybridation soit une paire stable, soit un mauvais appariement G-T appel de façon générale et dans ce qui suit «mismatch» G-T. Cette méthode a été appliquée avec succès pour la synthèse de cADN codant la relaxine de rat [P. Hudson et coll., Nature, (1981), 291, 127] mais est difficilement généralisable en raison de son manque de sensibilité notamment.

b) préparer toutes les séquences possibles avec les ambiguïtés correspondantes [B.E. Noys et coll., Proc. Natl. Acad. Sci., USA. (1979), 76, 1770 et M. Mevarech et coll., J. Biol. Chem., (1979), 254, 7472], ce qui peut nécessiter un énorme travail de synthèse.

c) synthétiser des «sondes mixtes» («mixed probes») avec toutes les ambiquïtés sur la même séquence. Cette technique a donné de bons résultas pour la β-globine [R.B. Wallace et coll., Nucleic Acids Research, (1981), 9, 879], par exemple, mais représente pour le chimiste organicien un côté «frustrant» vu la complexité du mélange obtenu et le nombre de trimères mis en jeu et au surplus ne donne pas toujours des résultats satisfaisants en raison notamment des réactivités différentes des différents trimères mis en jeu au niveau de chaque ambiguïté, ce qui a pour conséquence pratique que l'on n'a pas de certitude quant à la composition exacte du mélange obtenu.

L'invention remédie, au moins en partie, aux inconvénients des techniques antérieures en fournissant une sonde qui:

- peut à elle seule remplacer des mélanges complexes de séquences, ce qui correspond à un important gain de temps et d'argent;
- présente une composition bien définie;
- présente, par rapport notamment aux sondes définies sous a) ci-dessus, une meilleure sensibilité dans la détection après hybridation avec des séquences complémentaires et une meilleure stabilité de l'hybride ainsi formé (température de fusion plus élevée);

et au surplus, comme il sera vu plus loin,

- offre la possibilité d'être utilisée avec des méthodes d'identification faisant intervenir des réactions immunologiques voire immuno-enzymatiques à la place des détections radioactives relativement peu sensibles et présentant les inconvénients bien connus, utilisées jusqu'à présent.

On savait que lorsqu'on remplace dans une séquence nucléotidique une désoxy-2′ adénosine (A) par une amino-2 désoxy-2′ adénosine (A*), cela conduit, dans l'hybride formé par cette séquence, à la transformation d'une paire AT présentant deux liaisons hydrogène, en une paire A*T présentant trois liaisons hydrogène et qui a une stabilité équivalente à celle d'une paire CG [R.C. Comtor et P.R. Schimmel, Biophysical Chemistry, W.H. Freeman, San Franciso, (1980)].

Des séquences autocomplémentaires (hexamères et octamères) comportant des groupes amino-2 désoxy-2′ adénosine ont déjà été synthétisées pour des études spectroscopiques (dichroïsme circulaire), dans le but d'étudier la stabilité et la conformation (sous forme B ou Z) de modèles d'ADN [B.L. Gaffney et coll., Nucleic Acids Research, (1982), 10, 4351 et Tetrahedron, (1984), 40. 3].

Il n'avait cependant pas encore été proposé, ni même suggéré, de préparer des séquences d'oligonucléotides comportant des groupes adénine modifiés de telle sorte qu'ils soient susceptibles de former trois liaisons hydrogène avec les groupes réactifs de la thymine et utilisables en tant que sondes présentant les avantages de la sonde selon l'invention, décrits plus haut.

La sonde selon l'invention qui est constituée d'un fragment d'oligonucléotide de structure prédéterminée par l'application envisagée, est caractérisée en ce qu'une partie au moins des groupes adénine y est remplacée par des groupes adénine modifiés de telle sorte qu'ils soient susceptibles de former trois liaisons hydrogène avec les groupes réactifs de la thymine ou de l'uracile.

Les groupes adénine sont avantageusement modifiés par introduction, sur le sommet C-2 du cycle pyrimidine, d'un groupement susceptible de donner une liaison hydrogène avec l'atome d'oxygène fixé sur le sommet C-2 de la thymine ou de l'uracile.

L'invention a plus particulièrement pour objet une sonde constituée d'un fragment d'oligonucléotide de structure prédéterminée par l'application envisagée, caractérisée en ce qu'une partie au moins des groupes adénine y est remplacée par des groupes adénine modifiés par introduction, sur le sommet C-2 du cycle pyrimidine, d'un groupement susceptible de donner une liaison hydrogène avec l'atome d'oxygène fixé sur le sommet C-2 de la thymine ou de l'uracile.

Pour qu'une liaison hydrogène relativement stable de ce type puisse s'établir, il convient que le groupement introduit sur le groupe adénine soit le plus court possible. Des groupements préférés de ce type sont les groupements $-NH_2$, OH et -SH. Le groupement $-NH_2$ est particulièrement préféré.

Selon un mode préféré de réalisation, l'invention a pour objet une sonde constituée d'un fragment d'oligonucléotide de structure prédéterminée par l'application envisagée, caractérisée en ce qu'une partie au moins des groupes adénine y est remplacée par des groupes adénine modifiés par introduction, sur le sommet C-2 du cycle pyrimidine d'une groupement $-NH_2$, -OH ou -SH, de préférence $-NH_2$, c'est-à-dire des groupes représentés par la formule générale

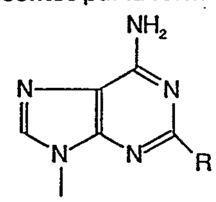

dans laquelle R représente $-NH_2$, -OH ou -SH.

La sonde selon l'invention peut, de façon particulièrement avantageuse, remplacer une «sonde mixte» telle que définie plus haut qui est déduite d'une séquence particulière d'aminoacides et comporte des ambiguïtés.

On a en effet découvert qu'on pouvait obtenir une sonde donnant une hybridation analogue à celle d'une sonde mixte et conférant une sensibilité analogue dans la détection après hybridation avec des séquences complémentaires, en réalisant une séquence déduite d'une séquence particulière d'aminoacides dans laquelle chaque dégénérescence est remplacée par l'un des nucléotides de la dégénérescence, notamment T pour une dégénérescence C/T et G pour une dégénérescence G/A, et au moins une partie des groupes adénine est remplacée par des groupes adénine modifiés tels que définis plus haut. Cette découverte est surprenante, notamment dans la mesure où, comme indiqué plus haut et comme il résulte clairement des essais comparatifs décrits plus loin, le choix de nucléotides particuliers dans les cas de dégénérescence peut conduire à des «mismatchs» qui ont pour conséquence un manque de sensibilité dans la détection, voire même une quasi-impossibilité d'utiliser la sonde obtenue.

Selon un mode particulièrement avantageux de réalisation, l'invention a donc pour objet une sonde telle que définie plus haut, caractérisée en ce qu'elle est constituée d'une séquence déduite d'une séquence particulière d'aminoacides et dans laquelle chaque dégénérescence est remplacée par l'un des nucléotides de la dégénérescence, notamment T pour une dégénérescence C/T et G pour une dégénérescence G/A.

Bien que cela ne soit pas indispensable, il est préférable dans tous les cas, et en particulier dans ce dernier mode de réalisation de l'invention, pour obtenir une meilleure sensibilité de détection, de remplacer tous les groupes adénine du fragment d'oligonucléotide par des groupes adénine modifiés tels que définis plus haut.

Pour la plupart des applications, la sonde selon l'invention comporte moins de 20 nucléotides. Toutefois, pour certaines applications particulières, il peut être souhaitable de disposer d'un fragment plus «long» d'oligonucléotide. Compte tenu de l'absence d'ambiguïtés, il n'existe en principe aucune limitation «technique» à la préparation de fragments plus longs. Ainsi, on a montré qu'il était possible d'obtenir des séquences comportant plus de 40 nucléotides.

La sonde selon l'invention peut être préparée selon tout procédé classique de préparation de fragments d'oligonucléotides en remplaçant une partie au moins des groupes acide adénylique ou acide désoxyadénylique, selon que l'on prépare un fragment d'ARN ou respectivement d'ADN, par des groupes acide adénylique ou respectivement désoxyadénylique comportant chacun un groupe adénine modifié tel que défini plus haut.

Avantageusement, la préparation est effectuée en phase solide essentiellement par fixation d'un monomère, dimère ou trimère par son extrémité 3' sur l'extrémité 5' du nucléotide terminal 3' de la séquence désirée, fixé sur un support solide, puis fixations successives de monomères, dimères et/ou trimères, à chaque fois sur le nucléotide terminal 5' de la chaîne obtenue, jusqu'à obtention de la fraction d'oligonucléotide désirée, avec séparation subséquente éventuelle du support.

Une telle préparation peut être effectuée selon les indications données par B.L. Gaffney et coll. dans les articles cités plus haut.

On entend ici par «monomère», «dimère» ou «trimère» des groupes constitués respectivement de 1, 2 ou 3 acides nucléiques, identiques ou différents, devant entrer dans la constitution de la sonde.

Il va de soi qu'au moment de la synthèse, les fonctions réactives de ces groupes dont la participation à la réaction envisagée n'est pas souhaitée, doivent être protégées au moyen de groupes protecteurs pouvant être ensuite éliminés dans des conditions non dégradantes pour la sonde obtenue.

A titre d'exemple non limitatif, la préparation

de monomères, dimères et trimères portant des groupes protecteurs de ce type peut être effectuée selon les indications de K. Itakura et coll. dans Nucleic Acids Research, (1980), 8, 5507.

Le déblocage des groupes protégés et la purification de l'oligonucléotide obtenu peuvent être effectués selon les indications de S. Tran Dinh et coll. dans Eur. J. Biochem. (1983), 133, 579–589 ou de R.W. Barnett et coll. dans Tetrahedron Lett. (1981), 22, 991–994.

Pour la préparation des monomères, dimères et trimères contenant un groupe adénine modifié, on utilise avantageusement un nucléotide préparé par phosphorylation du nucléotide correspondant, à savoir notamment, dans la série des ADN:

- lorsque le groupe adénine doit être modifié sur le sommet C-2 de la pyrimidine par un groupe $-NH_2$, la O-diméthoxytrityl-5′ N,N′diisobutyryl amino-2 désoxy-2′ adénosine préparée à partir de la désoxy-2′ guanosine, par analogie avec la technique décrite par W.L. Sung dans J.C.S. (Comm), 1089 (1981) en relation avec la thymidine;
- lorsque le groupe adénine doit être modifié sur le sommet C-2 de la pyrimidine par un groupe -OH, la O-diméthoxytrityl-5′ N-isobutyryl hydroxy-2 désoxy-2′ adénosine qui peut être préparée également par analogie avec la méthode de W.C. Sung, à partir du nucléoside dérivé de la xanthine correspondant et
- lorsque le groupe adénine doit être modifié sur le sommet C-2 de la pyrimidine par un groupe SH, la O-diméthoxytrityl-5′ N-isobutyryl mercapto-2 désoxy-2′ adénosine qui peut être préparée de la même manière que le dérivé hydroxy-2 correspondant, en remplaçant, selon une méthode connue, le groupe OH par un groupe SH.

Dans la série des ARN, on peut avantageusement utiliser les adénosines modifiées correspondantes.

Pour son utilisation, la sonde ainsi obtenue est avantageusement marquée, en vue de son repérage après hybridation, selon une technique usuelle dans le domaine des analyses biologiques.

Ainsi, elle peut être marquée radioactivement, notamment au $^{32}P$, au moyen de [$\gamma$-$^{32}P$]ATP en présence de polynucléotide kinase, comme décrit dans les exemples qui suivent.

Par ailleurs, et c'est là un des avantages particuliers des sondes selon l'invention, les demandeurs ont établi que la présence de groupes adénine modifiés, notamment par des groupes $-NH_2$ sur le sommet C-2 du cycle pyrimidine, favorise la configuration gauche, appelée configuration Z, des hybrides formés à partir des séquences de ce type, par rapport à la configuration usuelle B, et que dans la configuration Z, les groupes adénine modifiée sont plus accessibles à des réactifs extérieurs. Ceci permet leur repérage, notamment par des anticorps spécifiques de ces bases, de préférence monoclonaux, alors que la configuration B ne permet que difficilement, voire même empêché, l'accession des réactifs aux bases.

L'invention vise également une sonde telle que définie plus haut, marquée radioactivement, notamment par $^{32}P$.

L'invention vise encore les oligonucléotides comprenant des groupes protecteurs et correspondant aux sondes selon l'invention, éventuellement liés à des supports solides.

Elle comprend également les oligonucléotides du type susmentionné comprenant outre les groupes adénine modifiée utilisés selon la présente invention et les bases utilisées habituellement dans la synthèse d'oligonucléotides, d'autres bases dont la présence pourrait être jugée nécessaire, notamment des bases modifiées X et/ou Y, dont l'utilisation fait l'objet de la demande de brevet déposée le même jour par les mêmes demandeurs et ayant pour titre «sonde mixte et ses applications, son précurseur, leur préparation et dérivés comportant une base modifiée pour cette préparation».

Ces bases modifiées donnent au déblocage un mélange, notamment équimoléculaire selon les conditions de la réaction d'uracyle et de cytosine ou de thymine et de méthyl-5 cytosine d'une part, et de guanine et d'amino-2 adénine d'autre part, selon le schéma:

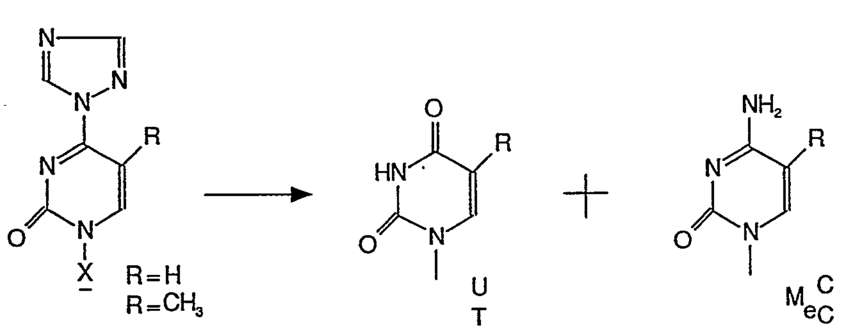

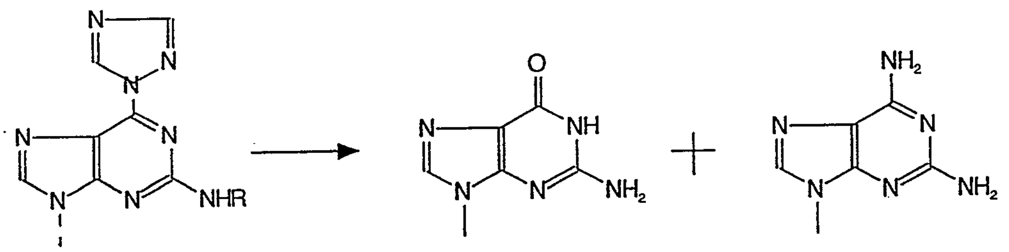

Y G A

L'invention comprend également les sondes obtenues au déblocage des oligonucléotides contenant les bases X et/ou Y.

La sonde selon l'invention peut donc être avantageusement utilisée dans des techniques d'analyse ou d'extraction, notamment d'ARN messagers ou d'ADN complémentaires, faisant appel à des mises en évidence comportant la formation de complexes immuns, notamment des réactions immuno-enzymatiques.

Il sera décrit ci-après, à titre d'exemple, l'application de l'invention à la résolution du problème posé par l'isolement de l'ADN complémentaire (cADN) de l'antithrombine III.

La séquence d'aminoacides prise en considération est

$^{251}$Met–Met–Tyr–Gln–Glu–Gly$^{256}$

ce qui correspond à la séquence d'acides ribonucléiques:

5'OH AUG AUG UA$^T_C$ CA$^A_G$ GA$^A_G$ G 3'$_{OH}$···

et à un cADN pouvant s'écrire:

5'OH ATG ATG TA$^T_C$ CA$^A_G$ CA$^A_G$ G 3'$_{OH}$···

et qui comprend 3 ambiguïtés.

On a préparé trois sondes destinées à s'hybrider avec ce cADN, à savoir:

1. une sonde mixte comportant les trois ambiguités correspondantes (mélange de 8 hexadécamères):

3' TAC TAC AT$^A_G$ GT$^C_T$ CT$^C_T$ C 5'.

cette sonde a été construite à partir de triplets en phase solide selon la technique de R.B. Wallace et coll. décrite dans Nucleic Acids Research (1981), 9, 879;

2. la séquence spécifique

3' TAC TAC AT*$^A_G$ GT*$^C_T$ CT*$^C_T$ C 5'

qui a été préparée à l'aide de dimères et trimères en phase solide selon la technique de H. Ito et coll., décrite dans Nucleic Acids Research (1982), 10, 1755, dans cette séquence, lorsqu'il y avait dégénérescence C/T on a choisi T, et en G/A on a choisi G, pour les raisons indiquées plus haut; dans ce choix, on a introduit un «mismatch» sur seize nucléotides (voir plus loin la structure réelle du cADN);

3. la séquence spécifique

3' T*AC T*AC *ATG GTT CTT C 5',

selon l'invention qui a été préparée à l'aide de monomères, dimères et trimères en phase solide selon les techniques indiquées plus haut en référence à ce type de séquençage; dans cette séquence, le choix de la dégénérescence est le même que sur la séquence n° 2 et on a remplacé l'adénosine A par l'amino-2 adénosine A*; on a montré que cette séquence forme, avec le cADN, un hybride plus stable que la séquence N° 2 (augmentation de la température de fusion de l'hybride) et donne une hybridation analogue à celle de la sonde mixte n° 1.

Ces trois sondes ont été marquées au $^{32}$P et ont servi à analyser une «banque» contenant le cADN de l'antithrombine III. Les sondes n$^{os}$ 1 et 3 ont permis d'isoler le cADN dont la structure a été prouvée par séquençage être:

5'OH ATGATGTACCAGGAAG 3'$_{OH}$

Dans cette formule, on a souligné d'un trait les nucléotides pour lesquels il y avait ambiguïté, mais sans «mismatch» pour les sondes n° 2 et 3 et de deux traits celui pour lequel il y a «mismatch» des sondes n' 2 et 3.

Partie expérimentale

Exemple préliminaire: Préparation du dérivé protégé d'acide désoxyadénylique modifié:

O-diméthoxytrityl-5' O-(chloro-2 phényl cyanoéthyl) phosphate-3' N,N'diisobutyryl amino-2 désoxy-2' adénosine (DMT $A^{2\text{-NHiBu}}_{iBu}$ P)

Dans un ballon séché à l'étuve, 312 mg (4,52 mmoles) de triazole sublimé sont dissous dans 4,5 ml de dioxanne anhydre. On ajoute 372 mg (1,50 mmoles) de 2-chlorophényl phosphorodichloridate et, à 0°C, 445 µl de triéthylamine anhydre. On obtient un épais précipité blanc qui est laissé sous agitation à température ambiante pendant une heure et demie. On filtre ce précipité qui est lavé avec du dioxanne directement sur 620 mg (0,94 mM) de O-diméthoxytrityl-5' N,N'diisobutyryl amino-2 désoxy-2' adénosine (DMT $A^{2\text{-NHiBu}}_{iBu}$) coévaporé 2 fois à la pyridine. On laisse sous agitation pendant une heure, puis on ajoute 160 µl de cyanoéthanol et 233 µl de N-méthyl imidazole. Deux heures après, la réaction est terminée, le produit est évaporé à sec, repris avec du dichlorométhane, lavé deux fois avec du $NaH_2PO_4$ à 5%, une fois avec du $NaHCO_3$ à 5% et une fois avec l'eau. La phase organique est séchée sur sulfate de sodium, filtrée et évaporée à sec. La mousse obtenue est précipitée à l'éther de pétrole, puis chromatographiée sur colonne de silice éluée au dichlorométhane enrichi progressivement en méthanol. On obtient 470 mg (rendement 47%) de nucléotide DMT $A^{2\text{-NHiBu}}_{iBu}$ P.

[CCM: 0,70 $CH_2Cl_2$–MeOH (90-10)]

Exemple 1

Synthèse de la sonde mixte n° 1

La sonde mixte a été «montée» à partir de 19 mg de résine T (résine portant le nucléotide T et préparée selon la technique de K. Itakura et coll. décrite dans Nucleic Acids Research (1980), 8, 5473) avec les trimères suivants préparés selon la technique de K. Itakura et coll. indiquée plus haut.

```
CTT  CTT  GAT
 +    +    +    ACA  TCA   T—
CCT  CCT  GGT
                                 Support solide
          ↑    ↑    ↑    ↑
Rendements: 86%  83%  79%  50%
```

Après le dernier couplage, la résine est traitée par 500 µl d'un mélange molaire de pyridine aldoxime (PAO) dans la tétraméthyl guanidine (TMG) additionné de 500 µl de TMG pendant une nuit. Après évaporation à sec et chauffage avec l'ammoniaque concentrée à 50°C pendant 3 heures, le milieu réactionnel est purifié par chromatographie sur une colonne de Sephadex G-10 (dénomination commerciale de la société Pharmacia pour une colonne de polysaccharide réticulé) suivie d'une chromatographie liquide haute performance (HPLC) sur colonne en phase inverse. Le mélange d'oligonucléotides est ensuite détritylé avec l'acide acétique à 80% pendant 5 mn, évaporé à sec (9 D.O.) (1 D.O. = une unité de densité optique, correspondant à 20–40 µg de séquence) et purifié par électrophorèse préparative sur gel d'acrylamide.

Exemple 2

Synthèse de la séquence n° 2

De même, la séquence n° 2 est synthétisée en phase solide à partir de 20 mg de résine T avec le triisopropyl-benzène sulfonyl nitro-triazole (TPSNT) comme agent de couplage

```
CTT  CTT  GGT  AC   AT   CA      T—
          ↑    ↑    ↑    ↑    ↑
Rendements: 61%  82%  74%  78%  96%
```

La séquence est purifiée comme décrit auparavant en relation avec l'exemple 1 (6,6 D.O.).

(avec A)

Exemple 3

Synthèse de la séquence n° 3 selon l'invention

La séquence contenant A* est synthétisée à partir de 30 mg de résine T

```
CTT          CTT  GGT  A*   C    A*   TC   A*       T—
              ↑    ↑    ↑    ↑    ↑    ↑    ↑
Rendements:  63%  98%  ≦99% 77%  ≦99% 72%  ≦99%
```

Le déblocage comporte une étape supplémentaire consistant en un traitement avec 1,87 ml d'éthylènediamine pendant 76 heures après le chauffage avec l'ammoniaque concentrée. Après purification par HPLC et électrophorèse, on obtient 2 D.O. de l'oligonucléotide.

Exemple 4

Marquage des sondes nos 1, 2 et 3 au $^{32}P$

On ajoute 50 µCi de [$\gamma$-$^{32}P$]ATP sur 100 pmoles de la sonde dans un volume de 4 µl de mix et 0,5 µl de polynucléotide kinase [activité 5–20 $10^3$ unités/ml, Boehringer Mannheim]. On laisse une demi-heure à 37°C [T. Maniatis et coll., Molecular Cloning, Cold Spring Harbor Lab. (1982)]. La réaction est stoppée par addition de 1,3 µl de tampon au bleu de bromophénol 100 mM d'EDTA, 50% de glycérol. Le produit marqué est purifié sur gel (0,4×30×40 cm) contenant 19 g de bisacrylamide, 19 g d'acrylamide et 10 ml de tris borate 1 M pour un volume total de 100 ml.

L'électrophorèse sous programmation: 200 V, 43 W, 40 mA, est arrêtée quand le bleu de bromophénol atteint le tiers de la longueur de la plaque. La bande radioactive est découpée et extraite pendant une nuit dans 2 ml d'eau.

Exemple 5

Hybridation

3 colonies contenant un cADN complet de l'$AT_{III}$ et 3 colonies contenant un cADN non relaté à l'$AT_{III}$ on été repiquées en 3 exemplaires sur boîtes de milieu complet avec 50 µg/ml d'ampicilline. Les colonies ont été transférées sur 3 filtres de type Whatman 541, amplifiées 20 heures sur boîtes contenant 250 µg/ml de chloramphénicol et préparées pour l'hybridation, selon la technique décrite par J.P. Gergen et coll. dans Nucleic Acids Research, (1979), 7, 2115. Les filtres ont été préhybridés 2 heures à 42°C en 6 NET (1 NET = 0,15 M NaCl, 0,015 M tris HCl, pH = 7,5, 0,001 M EDTA), 0,5% Monidet P40 (dénomination commerciale pour un détergent), 100 µg/ml de tRNA de levure et 100 µg/ml d'ADN soniqué de

sperme de saumon [technique de T. Maniatris et coll. dans l'article cité plus haut]. L'hybridation a été effectuée à 42°C pendant 20 heures dans la même solution en présence de $10^6$ cpm d'oligonucléotide marqué en 5' au [$\gamma$-$^{32}$ P]ATP, chaque filtre ayant été hybridé avec une des 3 sondes. Les filtres ont été lavés 4 fois 15 minutes à 40°C en 6 SSC (1 SSC = 0,15 M NaCl, 0,015 M citrate de sodium, pH = 7,2), 0,1% de dodécyl sulfate de sodium (SDS). Les filtres ont été soumis à une autoradiographie.

L'hybridation est visible sur la sonde mixte 1 (1 séquence sur 8 est complémentaire du cADN) et sur la séquence spécifique 3 comportant 3 A* (1 «mismatch» et 9/16 paires de type GC) alors que la séquence 2 (1 «mismatch» et 6/16 paires de type GC) ne s'hybride pas. La différence de température de fusion des hybrides avec les sondes n$^{os}$ 2 et 3 est de l'ordre de 7°C (l'hybride formé avec la sonde n° 3 ayant le point de fusion le plus élevé).

Il est rappelé que le «mix» est un mélange de:
- tris(hydroxyméthyl) aminométhane (500 mM, pH 8);
- $MgCl_2$ (100 mM); et
- dithiotréitol (50 mM).

## Revendications

1. Sonde de détection d'acides nucléiques codant pour un peptide de séquence connue en acides aminés et pouvant présenter des séquences nucléotidiques différentes en raison de la dégénérescence du code génétique, en particulier une ou plusieurs dégénérescences C/T (cytidine/thymidine) et/ou une ou plusieurs dégénérescences G/A (guanine/adénine), caractérisée en ce qu'elle comprend un oligonucléotide de séquence complémentaire de celle d'un fragment de l'acide nucléique à détecter, et comportant un motif T pour une dégénérescence C/T de l'acide nucléique et un motif G pour une dégénérescence G/A, une partie au moins des groupes adénines de l'oligonucléotide étant remplacée par des groupes adénines substitués, de manière à pouvoir établir trois liaisons hydrogène avec les groupes réactifs de la thymidine ou de l'uracile présents sur le fragment de l'acide nucléique.

2. Sonde de détection d'acides nucléiques codant pour un peptide de séquence connue en acides aminés et pouvant présenter des séquences nucléotidiques différentes en raison de la dégénérescence du code génétique, en particulier une ou plusieurs dégénérescences C/T (cytidine/thymidine) et/ou une ou plusieurs dégénérescences G/A (guanine/adénine), caractérisée en ce qu'elle comprend une séquence d'oligonucléotides complémentaire de celle d'un fragment de l'acide nucléique à détecter et comportant un motif T pour une dégénérescence C/T de l'acide nucléique et un motif G pour une dégénérescence G/A, une partie au moins des groupes adénine de l'oligonucléotide renfermant sur le sommet C-2 du cycle pyrimidine, un groupement susceptible de donner une liaison hydrogène avec l'atome d'oxygène fixé sur le sommet c-2 de la thymine ou de l'uracile du fragment de l'acide nucléique.

3. Sonde selon la revendication 2, caractérisée en ce que le groupe adénine modifié répond à la formule générale:

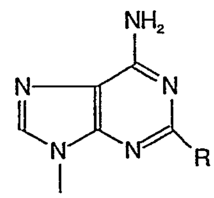

dans laquelle R représente $-NH_2$, -OH ou -SH.

4. Sonde selon l'une quelconque des revendications 1 à 3, caractérisée en ce que tous les groupes adénine sont des groupes adénine modifiés, de préférence des groupes amino-2 adénine.

5. Sonde selon l'une quelconque des revendications 1 à 4, caractérisée en ce qu'elle est marquée en vue de son repérage après hybridation, notamment radioactivement ou par une protéine, notamment un anticorps ou une enzyme.

6. Oligonucléotide éventuellement lié à un support solide et portant éventuellement des groupes protecteurs sur ses fonctions réactives comportant au moins un motif adénine modifié par un groupe -OH ou -SH, selon la revendication 3.

7. Procédé de préparation de la sonde selon l'une quelconque des revendications 1 à 5 ou de l'oligonucléotide selon la revendication 6, caractérisé en ce qu'il consiste essentiellement à effectuer, en phase solide, la fixation d'un monomère, dimère ou trimère par son extrémité 3' sur l'extrémité 5' du nucléotide terminal 3' de la séquence désirée, fixé sur un support solide puis à fixer successivement des monomères, dimères et/ou trimères, à chaque fois sur le nucléotide terminal 5' de la chaîne obtenue, jusqu'à obtention du fragment d'oligonucléotide désiré en vue de l'utilisation comme sonde, avec séparation subséquente éventuelle du support.

8. Procédé de détection de fragments d'ADN dans un échantillon, caractérisé en ce qu'on met en contact une sonde selon l'une quelconque des revendications 1 à 5, avec lesdits fragments dans des conditions permettant la formation d'une molécule duplex hybride dans laquelle le ou les motifs adénine de la sonde s'apparient avec la thymine des fragments d'ADN et forme trois liaisons hydrogène avec les groupes réactifs de la thymine, et on détecte la molécule duplex hybride.

9. Duplex d'ADN hybride comprenant une séquence d'ADN hybridée avec des sondes selon les revendications 1 à 5 ou un oligonucléotide selon la revendication 6, l'oligonucléotide étant éventuellement marqué.

**Patentansprüche**

1. Sonde zum Nachweis von Nucleinsäuren, die ein Peptid von bekannter Aminsäuresequenz codieren und aufgrund der Degeneration des genetischen Codes unterschiedliche Nucleotidsequenzen, insbesondere eine oder mehrere C/T (Cytidin/Thymidin)-Degenerationen und/oder eine oder mehrere G/A (Guanin/Adenin)-Degenerationen aufweisen können, dadurch gekennzeichnet, dass sie ein Oligonucleotid mit einer Sequenz darstellt, die derjenigen eines Fragments der nachzuweisenden Nucleinsäure komplementär ist und eine T-Gruppe für eine C/T-Degeneration der Nucleinsäure und eine G-Gruppe für eine G/A-Degeneration enthält, wobei wenigstens ein Teil der Adeningruppen des Oligonucleotids durch substituierte Adeningruppen ersetzt ist, so dass drei Wasserstoffbindungen mit den reaktionsfähigen Gruppen des Thymidins bzw. des Uracils am Nucleinsäurefragment gebildet werden können.

2. Sonde zum Nachweis von Nucleinsäuren, die ein Peptid von bekannter Aminsäuresequenz codieren und aufgrund der Degeneration des genetischen Codes unterschiedliche Nucleotidsequenzen, insbesondere eine oder mehrere C/T (Cytidin/Thymidin)-Degenerationen und/oder eine oder mehrere G/A (Guanin/Adenin)-Degenerationen aufweisen können, dadurch gekennzeichnet, dass sie eine Oligonucleotidsequenz darstellt, die derjenigen eines Fragments der nachzuweisenden Nucleinsäure komplementär ist und eine T-Gruppe für eine C/T-Degeneration der Nucleinsäure und eine G-Gruppe für eine G/A-Degeneration enthält, wobei wenigstens ein Teil der Adeningruppen des Oligonucleotids an der C-2-Spitze des Pyrimidinringes eine Gruppierung enthält, die mit dem auf der C-2-Spitze des Thymins oder des Uracils des Nucleinsäurefragments fixierten Sauerstoffatoms eine Wasserstoffbindung zu ergeben vermag.

3. Sonde nach Anspruch 2, dadurch gekennzeichnet, dass die modifizierte Adeningruppe der allgemeinen Formel

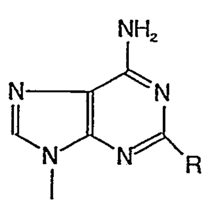

entspricht, worin R $-NH_2$, $-OH$ oder $-SH$ darstellt.

4. Sonde nach einem der Ansprüche 1 bis 3, dadurch gekennzeichnet, dass alle Adeningruppen modifizierte Adeningruppen, vorzugsweise Amino-2-Adeningruppen, sind.

5. Sonde nach einem der Ansprüche 1 bis 4, dadurch gekennzeichnet, dass sie zu ihrer Lokalisierung nach der Hybridisierung insbesondere radioaktiv oder durch ein Protein, insbesondere einen Antikörper oder ein Enzym markiert ist.

6. Gegebenenfalls mit einem festen Träger verbundenes Oligonucleotid, das gegebenenfalls an den reaktionsfähigen Funktionen, die wenigstens eine durch eine Gruppe $-OH$ oder $-SH$ modifizierte Adeningruppe nach Anspruch 3 enthalten, Schutzgruppen aufweist.

7. Verfahren zur Herstellung der Sonde nach einem der Ansprüche 1 bis 5 oder des Oligonucleotids nach Anspruch 6, dadurch gekennzeichnet, dass es im wesentlichen darin besteht, dass man in der Festphase am 5'-Ende des endständigen 3'-Nucleotids der gewünschten Sequenz, das auf einem festen Träger fixiert ist, ein Monomer, Dimer oder Trimer mit ihrem 3'-Ende fixiert, wonach man nacheinander Monomere, Dimere und/oder Trimere jeweils am endständigen 5'-Nucleotid der erhaltenen Kette fixiert, bis man das als Sonde verwendbare gewünschte Oligonucleotidfragment erhält, wonach man gegebenenfalls den Träger abtrennt.

8. Verfahren zum Nachweis von DNA-Fragmenten in einer Probe, dadurch gekennzeichnet, dass man eine Sonde nach einem der Ansprüche 1 bis 5 mit diesen Fragmenten unter Bedingungen in Kontakt bringt, welche die Bildung eines hybriden Duplexmoleküls ermöglichen, in dem sich die Adeningruppe(n) der Sonde jeweils mit dem Thymin der DNA-Fragmente verbindet bzw. verbinden und mit den reaktionsfähigen Gruppen des Thymins drei Wasserstoffbindungen bilden, und man das hybride Duplex-Molekül nachweist.

9. Hybrider DNA-Duplex, der eine DNA-Sequenz darstellt, die mit Sonden nach einem der Ansprüche 1 bis 5 oder mit einem Oligonucleotid nach Anspruch 6 hybridisiert sind, das gegebenenfalls markiert ist.

**Claims**

1. A probe for detecting nucleic acids encoding for a peptide of known amino acid sequence and possibly having nucleotidic sequences different because of the degeneracy of the genetic code, particularly one or several C/T degeneracies (cytitine/thymidine) and/or one or several G/A degeneracies (guanine/adenine), wherein said probe comprises an oligonucleotide having a sequence complementary to the one of an acid nucleic fragment to detect, and containing a T moiety for a C/T degeneracy of the nucleic acid and a G moiety for a G/A degeneracy, at least a part of the adenine groups of the oligonucleotide being replaced by the substituted adenine groups, so as to be able to form three hydrogen bonds with the reactive groups of thymin and uracil present on the fragment of the nucleic acid.

2. A probe for detecting nucleic acids encoding for a peptide of known amino acid sequence and possibly having nucleotidic sequences different because of the degeneracy of the genetic

code, particularly one or several C/T degeneracies (cytidine/thymidine) and/or one or several G/A degeneracies (guanine/adenine), wherein said probe comprises an oligonucleotide having a sequence complementary to the one of an acid nucleic fragment to detect, and containing a T moiety for a C/T degeneracy of the nucleic acid and a G moiety for a G/A degeneracy, at least a part of the adenine groups of the oligonucleotide containing on the C-2 position of the pyrimidine cycle, a group capable of forming an hydrogen bond with the oxygen atom fixed to the C-2 position of thymine or of uracil of the acid nucleic fragment.

3. A probe according to claim 2, wherein the modified adenine group has the general formula:

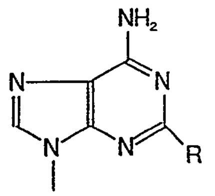

in which R represents $-NH_2$, $-OH$ or $-SH$.

4. A probe according to anyone of claims 1 to 3, wherein all the adenine groups are modified adenine groups, preferably 2-amino adenine groups.

5. A probe according to anyone of claims 1 to 4, characterized in that it is labelled in view of its location after hybridization, particularly radioactively or by a protein, especially an antibody or an enzyme.

6. An oligonucleotide optionally bound to a solid support and optionally having protective groups on its reactive functions comprising at least an adenine moiety modified by an $-OH$ or $-SH$ group according to claim 3.

7. A process for making a probe according to anyone of claims 1 to 5 or an oligonucleotide according to claim 6, essentially comprising fixing, in the solid phase, a monomer, dimer or trimer by the terminal 3′ to the terminal 5′ of the terminal 3′ nucleotide of the desired sequence, fixed to the solid support, then successively fixing monomers, dimers and/or trimers, each time to the terminal 5′ nucleotide of the chain obtained, until the production of the desired oligonucleotide fraction, in view of its use as a probe, with the possible subsequent separation of the support.

8. A process for detecting DNA fragments in a sample, comprising contracting a probe according to anyone of claims 1 to 5, with said fragments under conditions to form a hybrid duplex molecule in which the adenine moiety(ies) in said probe pairs with thymine in said DNA fragments and forms three hydrogen bonds with the reactive groups of thymine, and detecting the hybrid duplex molecule.

9. Hybrid DNA duplex comprising a DNA sequence hybrid with probes according to claims 1 to 5 or an oligonucleotide according to claim 6, said oligonucleotide being optionally labelled.